# EUROPÄISCHE PATENTSCHRIFT

(11) **EP 1 813 305 B1**
(45) Veröffentlichungstag und Bekanntmachung des Hinweises auf die Patenterteilung: **11.05.2011**
(21) Anmeldenummer: 07000837.0
(22) Anmeldetag: 17.01.2007
(51) Int. Cl.: A61M 39/24

(54) **Rückschlagventil, insbesondere für die Medizintechnik**
Non-return valve, in particular for medical engineering
Clapet anti-retour, en particulier pour la technique médicale

(30) Priorität: 30.01.2006 DE 202006001475 U
(43) Veröffentlichungstag der Anmeldung: 01.08.2007
(73) Patentinhaber: Filtertek B.V., County of Limerick (IE)
(72) Erfinder: Mijers, Jan Willem Marinus, 2014 AL Haarlem (NL)
(74) Vertreter: Beck, Alexander

(56) Entgegenhaltungen:
- WO-A-03/092788
- DE-A1- 4 331 718
- DE-U1-202004 013 704
- FR-A- 2 666 745

## Beschreibung

Die Erfindung betrifft ein Rückschlagventil, insbesondere für die Medizintechnik, mit einem ersten und einem mit diesem verbundenen zweiten Schlauchanschlussgehäuse und mit einer zwischen den beiden Schlauchanschlussgehäusen angeordneten Membran aus einem flexiblen Werkstoff, welche gegen einen ringförmigen Ventilsitz vorgespannt ist, und welche bei Überdruck in einem Eingangskanal des ersten Schlauchanschlussgehäuses unter Freigabe eines Durchflussquerschnittes von dem Ventilsitz abhebbar ist und bei Überdruck in einem Ausgangskanal des zweiten Schlauchanschlussgehäuses zum Schließen sicher und in Minimalzeiten auf den Ventilsitz andrückbar ist, wobei die Membran durchgehend ohne Öffnungen ausgebildet ist und auf beiden Seiten der Membran je ein ringförmiger beziehungsweise kreisförmiger Ventilraum vorgesehen ist, wobei der ringförmige Ventilraum den Ventilsitz umgibt und die beiden Ventilräume durch Bypasskanäle miteinander verbunden sind.

Ein derartiges Rückschlagventil ist nach einem früheren Vorschlag der Anmelderin aus der DE 105 45 421 C2 bekannt. Bei diesem bekannten Rückschlagventil ist die Membran als kreisförmige Scheibe aus einer Bahn aus Flüssigsilïkon, Silikon oder Naturgummi ausgestanzt und mit ihrem Randbereich an ihrem Umfang zwischen den beiden Schlauchanschlussgehäusen eingespannt. Rückschlagventile dieser Art werden vorzugsweise in Infusionsgeräten und dergleichen in der Medizintechnik eingesetzt. Hierbei fließt die Infusionsflüssigkeit aus einer Infusionsflasche in einen Tropfbehälter und von dort durch den Schlauch in einen Proportionalregler, der auch durch ein Rückschlagventil ersetzt werden kann. Anschließend an den Proportionalregler folgt noch ein auf den Infusionsschlauch aufgesetzter Regler in Form einer sogenannten Rollenklemme. Nach der Rollenklemme wird der Infusionsschlauch wie üblich über einen Klebeverband mit einer Nadel in die Vene des Patienten geführt. Das bekannte Ventil arbeitet ausgesprochen zufriedenstellend, bietet jedoch hinsichtlich der Produktion gewisse Probleme. Derartige Rückschlagventile werden zu Tausenden gefertigt und können daher aus Kostengründen nicht von Hand montiert werden. Es kommt daher ausschließlich eine automatische Montage in Betracht, welche von der Werkstoffseite und der Gestaltung bestimmte Voraussetzungen verlangt. Auf Grund der Tatsache, dass bei diesem bekannten Ventil die scheibenförmige Membran zwischen den beiden Schlauchanschlussgehäusen an ihrem Umfang eingeklemmt ist, ist der Ventilsitz, welcher einstückig mit einem der Schlauchanschlussgehäuse hergestellt ist, vor der Montage vergleichsweise frei zugänglich und kann daher beschädigt werden. Insbesondere werden ja die beiden Schlauchanschlussgehäuse durch Spritzgießen hergestellt, so dass bereits beim Auswerfen aus der Form Beschädigungen des Ventilsitzes vorkommen können, was dann zur Produktion von Ausschuss führt.

Ein Rückschlagventil gemäss dem Oberbegriff von Anspruch 1 ist aus dem Dokument WO 03/092788 A bekannt.

Ausgehend von diesem bekannten Ventil der eingangs definierten Art liegt der Erfindung daher die Aufgabe zugrunde, eine Ventilkonstruktion vorzuschlagen, welche es ermöglicht, den Ventilsitz in einen gegen Beschädigungen geschützten Bereich zu verlegen.

Erfindungsgemäß wird diese Aufgabe im wesentlichen dadurch gelöst, dass die Membran topfartig mit gegenüber ihrem Boden verstärkter Seitenwand ausgebildet ist, dass die Membran mit ihrer Seitenwand unter Vorspannung über eine ringförmige Stützwand geschoben ist, und dass der ringförmige Ventilsitz radial innerhalb der Stützwand angeordnet ist und axial der Stützwand gegenüberliegend auf der Außenseite des Bodens anliegt.

Es ist offensichtlich, dass aufgrund der Tatsache, dass es hierbei nicht mehr notwendig ist, die Membran zwischen den Schlauchanschlussgehäusen einzuklemmen, sondern dass diese durch die ringförmige Stützwand getragen wird, nunmehr der Ventilsitz in einen geschützten Bereich des zugehörigen Schlauchanschlussgehäuses verlegt werden kann, so dass Beschädigungen des Ventilsitzes insbesondere beim Auswerfen aus der Form oder bei der Montage ausgeschlossen werden. Die Montage des Ventils selbst wird ebenfalls erleichtert, indem die topfartige Membran zunächst mit ihrer verstärkten Seitenwand über die Stützwand des einen Schlauchanschlussgehäuses geschoben wird, und danach die Schlauchanschlussgehäuse miteinander verbunden und verschweißt werden können.

Eine besonders vorteilhafte Ausführungsform nach der Erfindung kann dadurch geschaffen werden, dass die Bypasskanäle zwischen der Seitenwand der Membran und dem zweiten Schlauchanschlussgehäuse vorgesehen sind. Diese Ausführungsform bietet den Vorteil, dass die zum Spritzgießen verwendeten Formen einfach und daher kostengünstig ausgeführt werden können, indem beispielsweise die Bypasskanäle in Axialrichtung des Schlauchanschlussgehäuses zwischen Stegen verlaufen, die bei fertig montiertem Ventil an der Seitenwand der Membran anliegen.

Folglich besteht eine vorteilhafte Weiterbildung dieser Ausführungsform darin, dass die Bypasskanäle in dem zweiten Schlauchanschlussgehäuse axial zur Seitenwand verlaufend ausgebildet sind.

Eine abgewandelte,' ebenfalls vorteilhafte Ausführungsform nach der Erfindung kann dadurch geschaffen werden, dass die Bypasskanäle unmittelbar in der Seitenwand der Membran selbst ausgebildet sind. Hierdurch ist ebenfalls eine einfache Gestaltung der Spritzgießform möglich, indem die an die Membran anliegende Seitenwand des Schlauchanschlussgehäuses glatt zylindrisch ausgebildet werden kann und die parallel und axial zur Seitenwand verlaufenden Bypasskanäle als einfache Nuten in der Seitenwand der Membran ausgeformt werden.

Im Einzelnen ist es von Vorteil, dass der den Ventilsitz umgebende Ringraum auf der Außenseite des Bodens der Membran über die Bypasskanäle mit dem Auslasskanal des Rückschlagventils verbunden ist.

Eine besonders vorteilhafte Ausführungsform nach der Erfindung kann dadurch geschaffen werden, dass die Stützwand mit dem zweiten Schlauchanschlussgehäuse einstückig ausgebildet ist. Hierdurch wird die oben erwähnte Erleichterung der Montage gewährleistet, indem ein einfaches Aufschieben der topfartigen Membran auf die Stützwand ermöglicht wird.

Bei einer vorteilhaften Weiterbildung nach der Erfindung ist es vorgesehen, dass die Schlauchanschlussgehäuse tief ineinander geschachtelt ausgebildet sind, derart, dass der Ventilsitz tief innerhalb des ersten Schlauchanschlussgehäuses liegt. Dieses Merkmal dient dem absoluten Schutz des Ventilsitzes gegen Beschädigungen bei der Fertigung und bei der Montage.

Im Einzelnen kann es von besonderem Vorteil sein, dass die Membran aus einem thermoplastischen Elastomer besteht. Dies wird durch die topfartige Konstruktion der Membran möglich und stellt einen erheblichen wirtschaftlichen Faktor dar, da die üblichen Membranen sehr aufwendig aus Silikon mit speziellen Werkzeugen hergestellt werden müssen.

Im Folgenden wird die Erfindung anhand von den in den Zeichnungen beispielhaft veranschaulichten Ausführungsformen näher erläutert. Es zeigt:
**Figur 1** eine stark vergrößerte geschnittene perspektivische Darstellung einer ersten Ausführungsform des Rückschlagventils;
**Figur 2** eine auseinandergezogene Darstellung der Einzelteile der Ausführungsform gemäß Figur 1,
**Figur 3** eine vergrößerte perspektivische Ansicht der Membran der ersten Ausführungsform;
**Figur 4** eine Figur 1 entsprechende Darstellung einer zweiten Ausführungsform eines Rückschlagventils nach der Erfindung;
**Figur 5** eine perspektivische Darstellung der Einzelteile des Ventils gemäß Figur 5 in auseinandergezogener Darstellung;
**Figur 6** eine stark vergrößerte perspektivische Ansicht der Membran der zweiten Ausführungsform gemäß den Figuren 4 und 5 von der Oberseite her;
**Figur 7** eine Figur 6 entsprechende perspektivische Darstellung der Membran von der Unterseite her;
**Figur 8** eine Figur 1 beziehungsweise Figur 4 entsprechende Darstellung einer dritten Ausführungsform eines Ventils nach der Erfindung;
**Figur 9** die Einzelteile des Ventils gemäß Figur 8 in auseinandergezogener Darstellung;
**Figur 10** eine stark vergrößerte Schnittansicht der Membran des Ventils gemäß den Figuren 8 und 9;
**Figur 11** eine Figur 1 entsprechende Darstellung einer weiteren Ausführungsform eines Rückschlagventils nach der Erfindung;
**Figur 12** die Einzelteile des Rückschlagventils gemäß Figur 11 in auseinandergezogener Darstellung;
**Figuren 13****,** **14** **und** **15** geschnittene perspektivische Darstellungen von drei weiteren Ausführungsformen des Rückschlagventils nach der Erfindung.

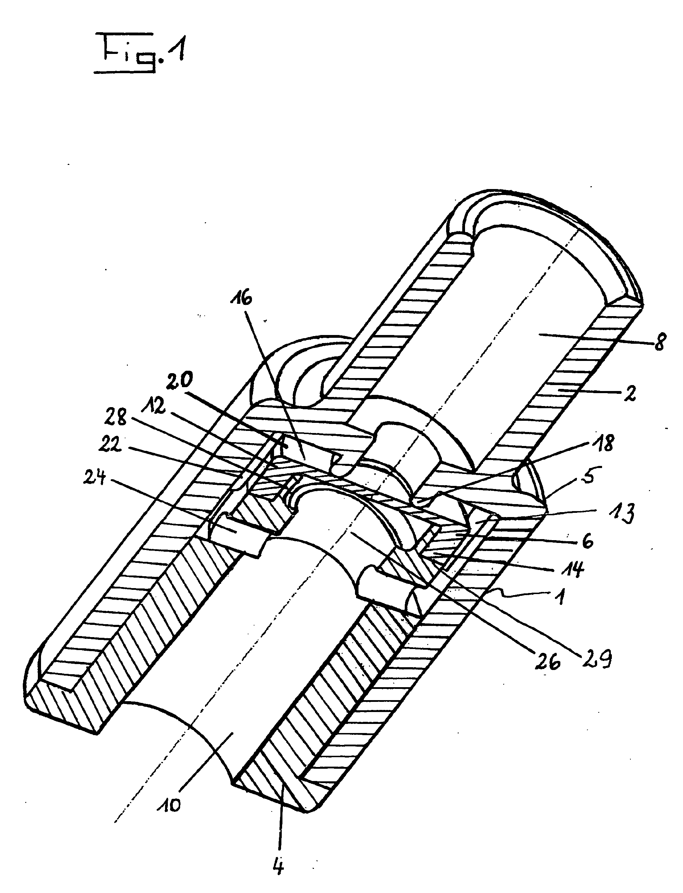

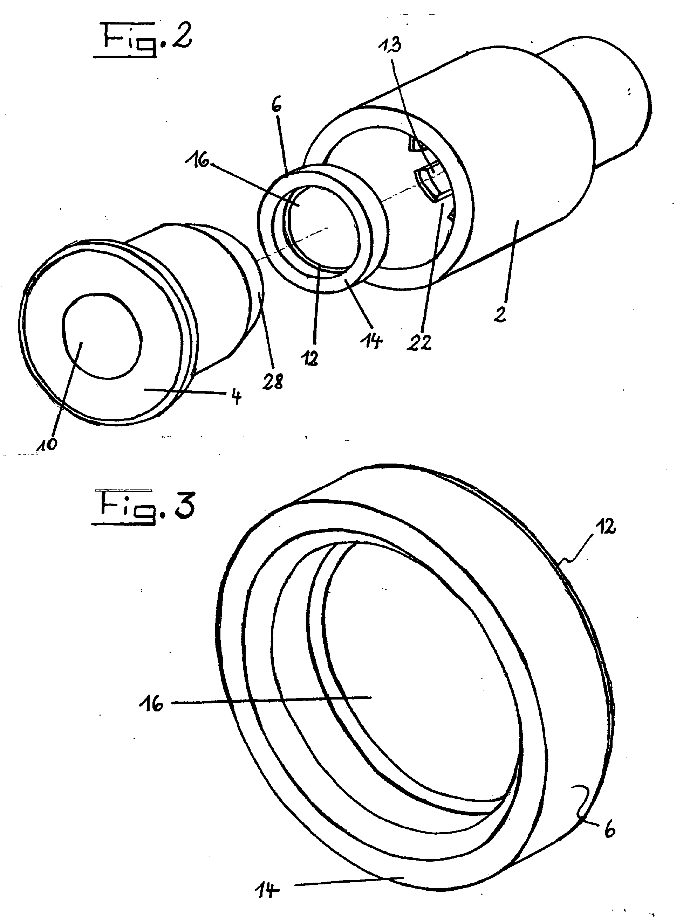

Es wird zunächst auf die Ausführungsform gemäß den Figuren 1 bis 3 Bezug genommen.

Das hier dargestellte Rückschlagventil 1 weist ein erstes Schlauchanschlussgehäuse 2 und ein zweites Schlauchanschlussgehäuse 4 auf, welche durch Spritzgießen aus Kunststoff hergestellt sind und miteinander durch beispielsweise Ultraschallschweißen verbunden sind. Die beiden Schlauchanschlussgehäuse 2 und 4 bilden zusammen das Ventilgehäuse 5. Zwischen den beiden Schlauchanschlussgehäusen 2 und 4 ist eine Membran 6 aus einem flexiblen Kunststoff angeordnet, welche einen Eingangskanal 8 des ersten Schlauchanschlussgehäuses 2 von einem Auslasskanal 10 des zweiten Schlauchanschlussgehäuses 4 trennt.

Wie gezeigt, ist die Membran 6 in ihrer Form topfartig ausgebildet, wobei die Seitenwand 12 der Membran 6 in ihrem Querschnitt stark vergrößert oder verstärkt ist. Der Boden 16 der Membran 6 ist in der für derartige Membranen üblichen Wandstärke dünn ausgebildet und ist durchgehend ohne Öffnungen geformt.

Dem Boden 16 der Membran gegenüberliegend ist ein ringförmiger Ventilsitz 18 einstückig mit dem ersten Schlauchanschlussgehäuse 2 ausgebildet und liegt gegen den Boden 16 an.

Den Ventilsitz 18 umgebend ist ein ringförmiger Ventilraum 20 vorgesehen, welcher über parallel zur Seitenwand 12 der Membran 6 verlaufende Bypasskanäle 22 und Verbindungskanäle 24 mit einem kreisförmigen Ventilraum 26 verbunden ist, welcher mit dem Auslasskanal 10 in Verbindung steht.

Wie insbesondere aus Figur 1 ersichtlich, ist die Seitenwand 12 der Membran 6 über eine ringförmige Stützwand 28 gestülpt und sitzt mit ihrer Unterkante 14 auf eine die Stützwand 28 umgebende Ringschulter 29 auf, wobei die Stützwand 28 einstückig mit dem zweiten Schlauchanschlussgehäuse 4 ausgebildet ist.

Wie gezeigt, verlaufen die Bypasskanäle 22 parallel zur Seitenwand 12 der Membran 6 beziehungsweise der zylindrischen Seitenwand des ersten Schlauchanschlussgehäuses und sind durch einstückig mit dem ersten Schlauchanschlussgehäuse ausgeformte Stege 13 voneinander getrennt, welche dichtend an der Seitenwand 12 der Membran 6 anliegen.

Wie insbesondere aus Figur 1 ersichtlich, sitzt der Ventilsitz 18 tief innerhalb des ersten Schlauchanschlussgehäuses 2 und ist folglich gegen jegliche Beschädigungen bei der Herstellung oder Montage geschützt. Dies wird durch die besondere Konstruktion der Membran 6 mit der ringförmigen Stützwand 28 ermöglicht, da bei der Montage der beiden Schlauchanschlussgehäuse zunächst die Membran 6 mit ihrer Seitenwand 12 über die Stützwand 28 unter Vorspannung gestülpt wird, und anschließend das zweite Schlauchanschlussgehäuse 4 in das erste Schlauchanschlussgehäuse 2 hineingeschoben wird, bis die Membran 6 den Ventilsitz 18 erreicht, wonach dann die beiden Schlauchanschlussgehäuse 2 und 4 miteinander verbunden werden.

Bei einem Überdruck in dem Einlasskanal 8 des ersten Schlauchanschlussgehäuses 2 wird der Boden 16 der Membran 6 von dem Ventilsitz 18 abgehoben und gibt einen Durchflussquerschnitt frei, so dass das Medium in den ringförmigen Ventilraum 20 und von dort durch die Bypasskanäle 22 und den Verbindungskanal 24 in den kreisförmigen Ventilraum strömen kann, und von dort durch den Auslasskanal 10 nach außen. Bei einem Überdruck in dem Ausgangskanal 10 des zweiten Schlauchanschlussgehäuses 4 schließt der Boden 16 der Membran 6 sicher gegen den Ventilsitz 18, indem er gegen diesen in Minimalzeiten angedrückt wird.

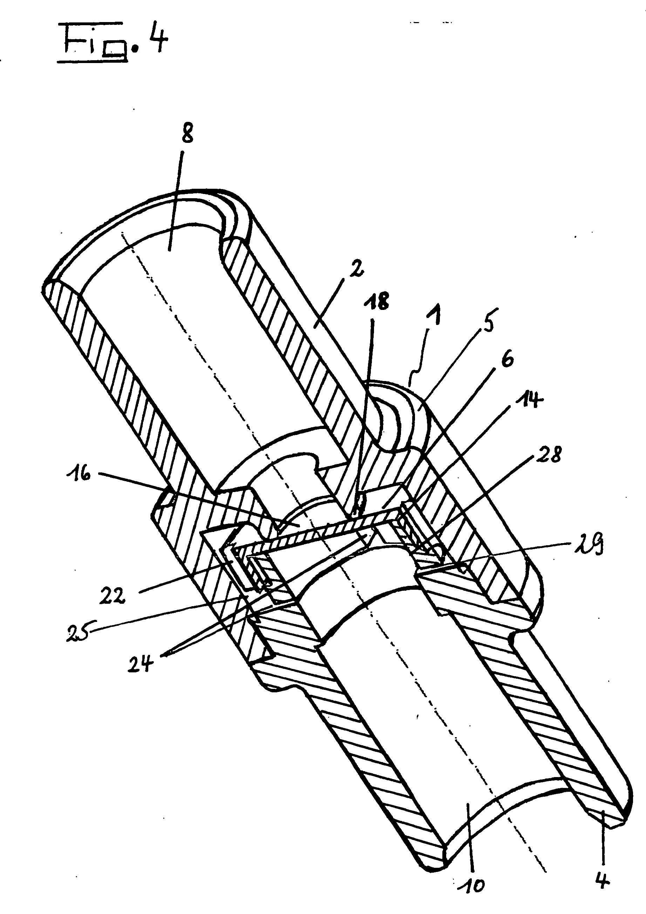

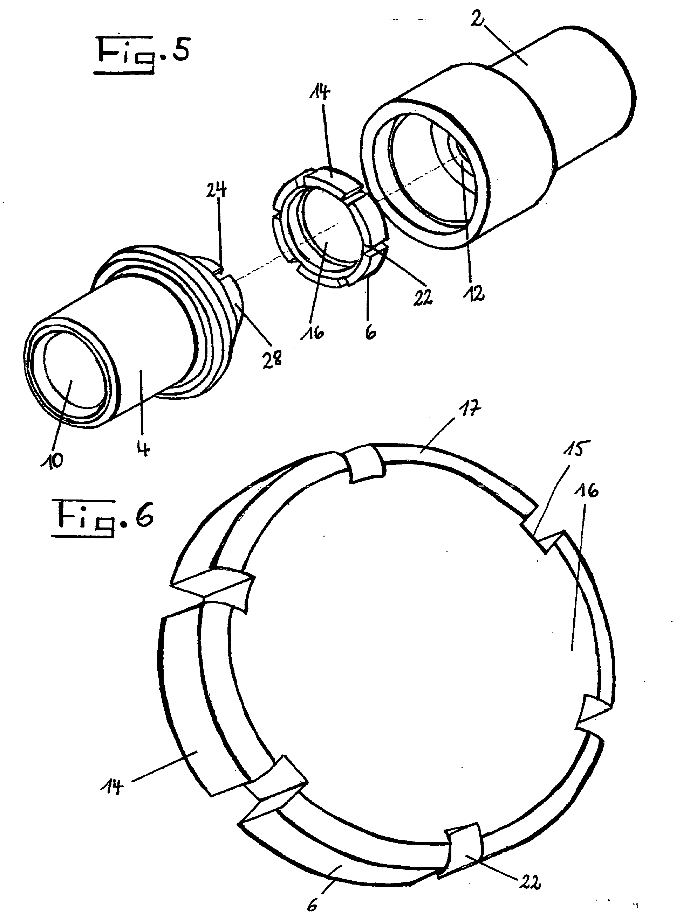

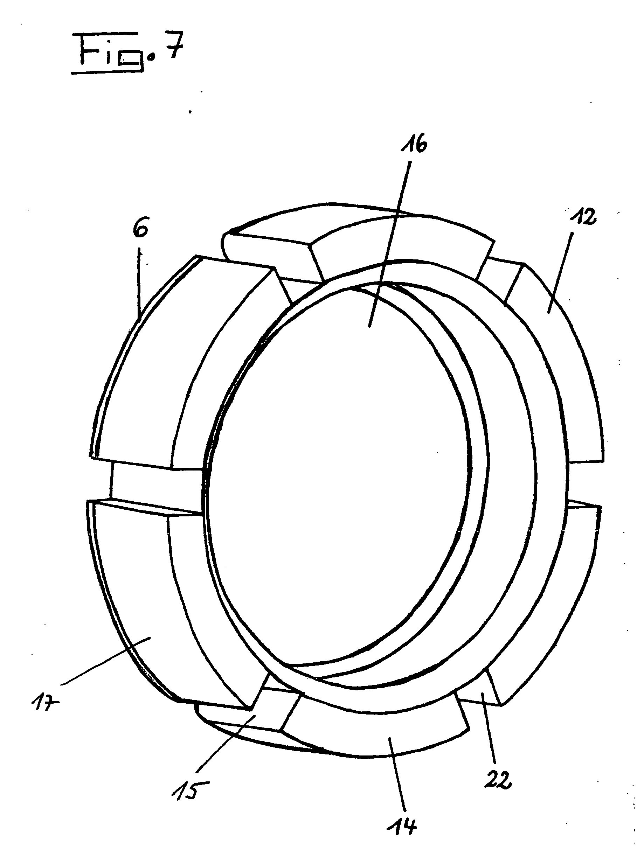

Die in den Figuren 4 bis 7 veranschaulichte Ausführungsform des Rückschlagventils nach der Erfindung entspricht im Wesentlichen der oben beschriebenen Ausführungsform, wobei für gleiche beziehungsweise gleichwirkende Teile gleiche Bezugszeichen verwendet wurden.

Gegenüber der ersten Ausführungsform unterscheidet sich die in den Figuren 4 bis 7 veranschaulichte Ausführungsform hinsichtlich der Ausgestaltung der Bypasskanäle.

Wie gezeigt, sind bei dieser Ausführungsform die Bypasskanäle 22 in der verstärkten Seitenwand 12 der Membran 6 als senkrecht zur Seitenwand 12 verlaufende Nuten 15 ausgebildet, wobei die zwischen den Nuten 15, welche die Bypasskanäle 22 bilden, liegenden Materialstreifen 17 in dem fertigmontierten Zustand gemäß Figur 4 an der zylindrisch und glatt ausgebildeten Innenseite der Seitenwand 11 des ersten Schlauchanschlussgehäuses anliegen.

Derartige, als Nuten 15 ausgebildete Bypasskanäle 22 in der Seitenwand 12 der Membran 6 können ohne Schwierigkeiten hergestellt werden, unabhängig davon, ob die Membran selbst aus Silikon oder aus einem thermoplastischen Elastomer hergestellt ist.

Bei der Ausführungsform gemäß Figuren 4 bis 7 ist insbesondere aus Figur 4 ersichtlich, dass hier die Verbindungskanäle 24 unterhalb der Ringschulter 29 der Stützwand 28 vorgesehen sind, so dass hier vor dem kreisförmigen Ventilraum 26 ein weiterer Ringraum 25 ausgebildet ist, in welchen die Bypasskanäle 22 münden. Der zweite Ringraum 25 ist daher über die Verbindungskanäle 24 mit dem kreisförmigen Ventilraum 26 verbunden.

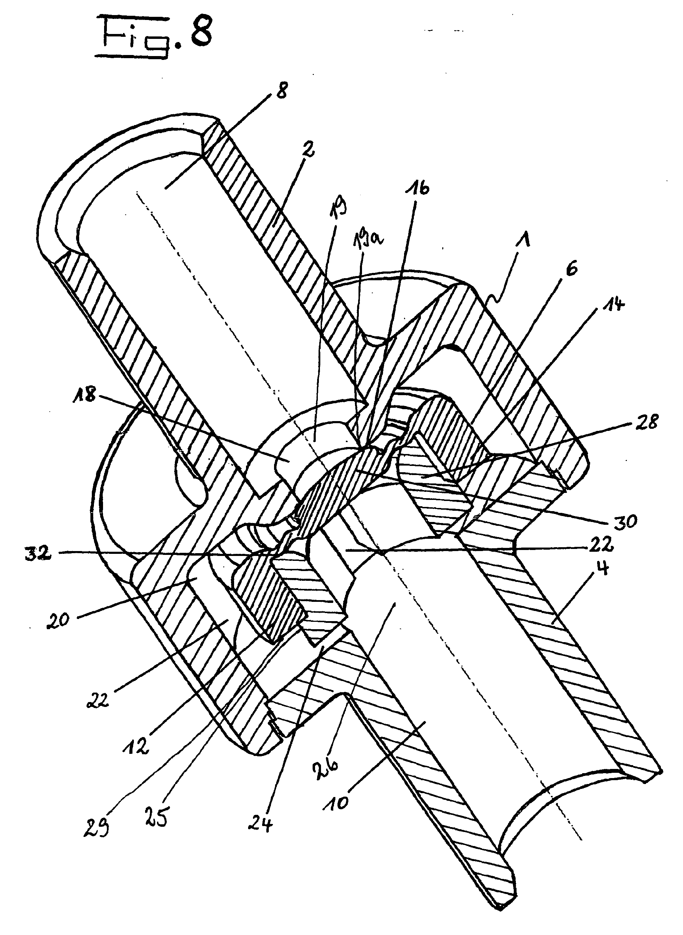

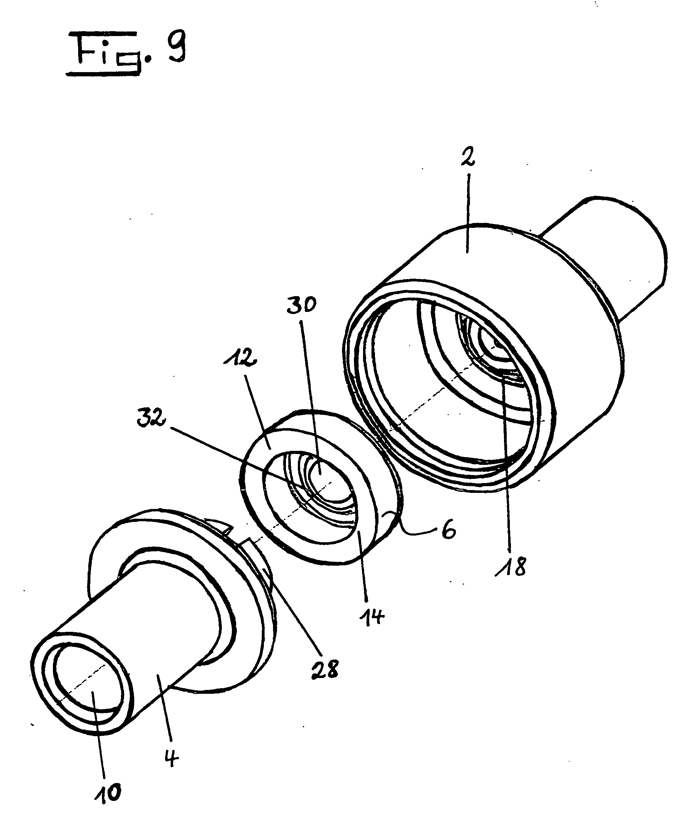

Die Ausführungsform gemäß den Figuren 8 bis 10 entspricht in wesentlichen Einzelheiten der in den Figuren 4 bis 7 veranschaulichten Ausführungsform, wobei jedoch bei dieser Ausführungsform die Membran 6 abweichend gestaltet ist. Die Bypasskanäle 22 sind in diesem Fall als um die Seitenwand 12 der Membran 6 verlaufender Ringkanal ausgebildet, wobei die Verbindungskanäle 24 wiederum den zweiten Ringraum 25 mit dem kreisförmigen Ventilraum 26 verbinden.

Der Ventilsitz 18 ist hierbei als einfache Bohrung 19 ausgebildet, deren Unterkante 19a den eigentlichen Ventilsitz bildet.

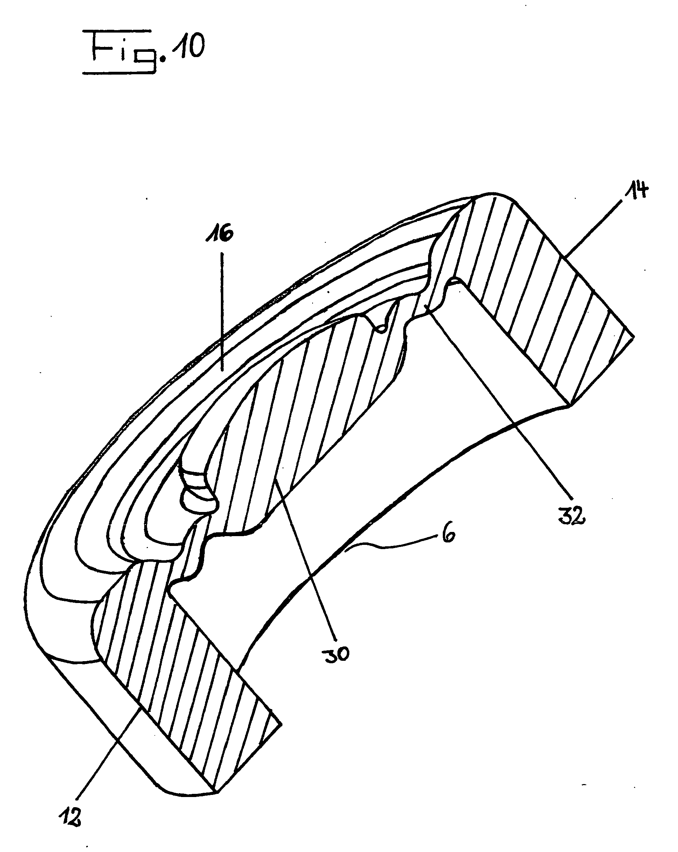

Wie insbesondere aus Figur 10 ersichtlich, ist bei dieser Ausführungsform die Membran 6 mit einer in der Mitte liegenden, linsenartigen Verdickung 30 versehen, welche über einen Verbindungsabschnitt 32 ringsum radial mit den Seitenwänden 12 verbunden ist. Der Verbindungsabschnitt 32 bildet quasi ein Gelenk für die Hub- und Absenkbewegung der das Schließglied des Ventils 1 bildenden linsenförmigen Verdickung 30.

Der Vorteil dieser Ausführungsform liegt in dem sehr einfachen Aufbau des Ventilsitzes, so dass hier die Formkosten für die Spritzgießform geringer sind. Wie bei sämtlichen anderen Ausführungsformen liegt auch hier der Ventilsitz 18 tief im Inneren des ersten Schlauchanschlussgehäuses 2 und ist gegen Beschädigungen bei der Herstellung und Montage absolut geschützt.

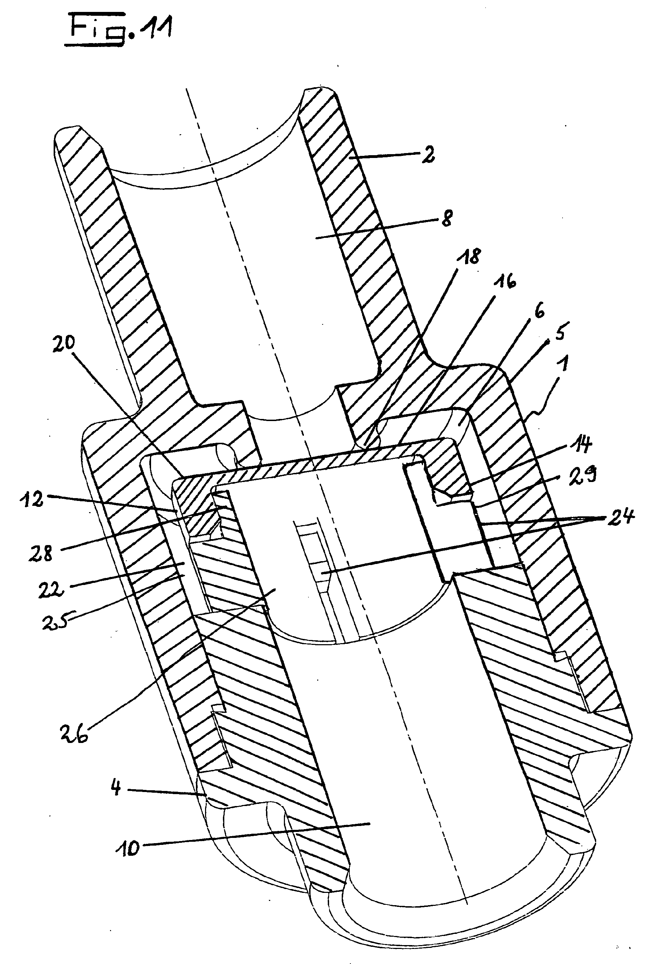

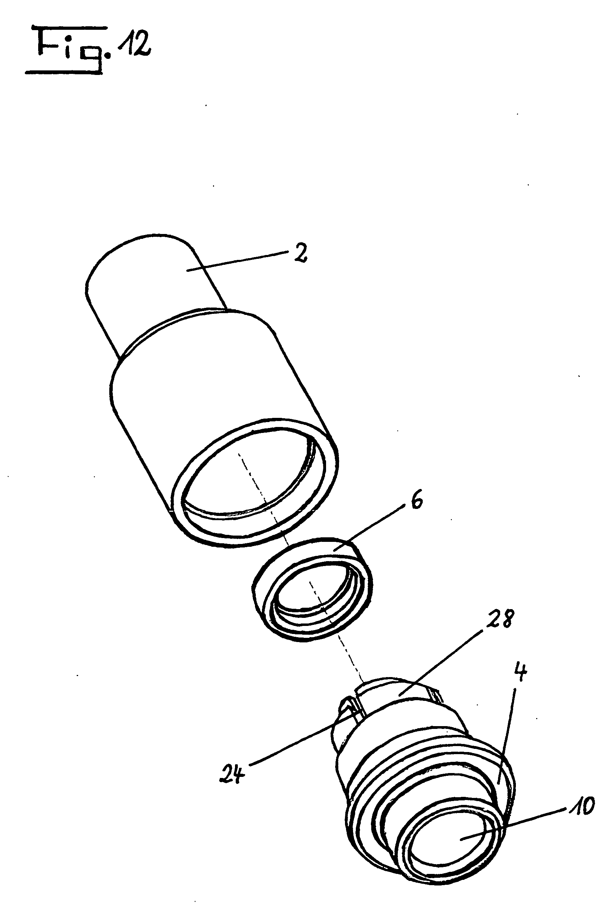

Die in den Figuren 11 und 12 veranschaulichten Ausführungsformen eines Rückschlagventils nach der Erfindung entspricht in wesentlichen Einzelheiten der Ausführungsform gemäß den Figuren 1 bis 3, so dass hinsichtlich weiterer Einzelheiten auf die oben stehende Beschreibung zu diesen Figuren verwiesen werden kann.

Abweichend entspricht hier der Außendurchmesser der Seitenwand der Membran 6 dem Außendurchmesser der Ringschulter 29 der Stützwand 28, so dass die Bypasskanäle 22 durch den die Seitenwand 12 umgebenden ringförmigen Raum gebildet sind, und wiederum in einen zweiten Ringraum 25 unterhalb der Ringschulter 29 führen. Wie aus Figur 11 ersichtlich, sind die Verbindungskanäle 24, welche den zweiten Ringraum 25 mit dem kreisförmigen Ventilraum 26 verbinden, als axiale Schlitze unterhalb der Ringschulter 29 ausgebildet.

Um die Spritzgießform für das zweite Schlauchanschlussgehäuse 4 möglichst einfach gestalten zu können, sind hierbei die Verbindungskanäle 24 als axiale Schlitze ausgebildet, die von der Oberkante der Stützwand 28 ausgehen, so dass, wie es dem Fachmann offensichtlich sein dürfte, sowohl das erste als auch das zweite Schlauchanschlussgehäuse 2, 4 mittels Formen herstellbar sind, die lediglich eine Trennebene aufweisen.

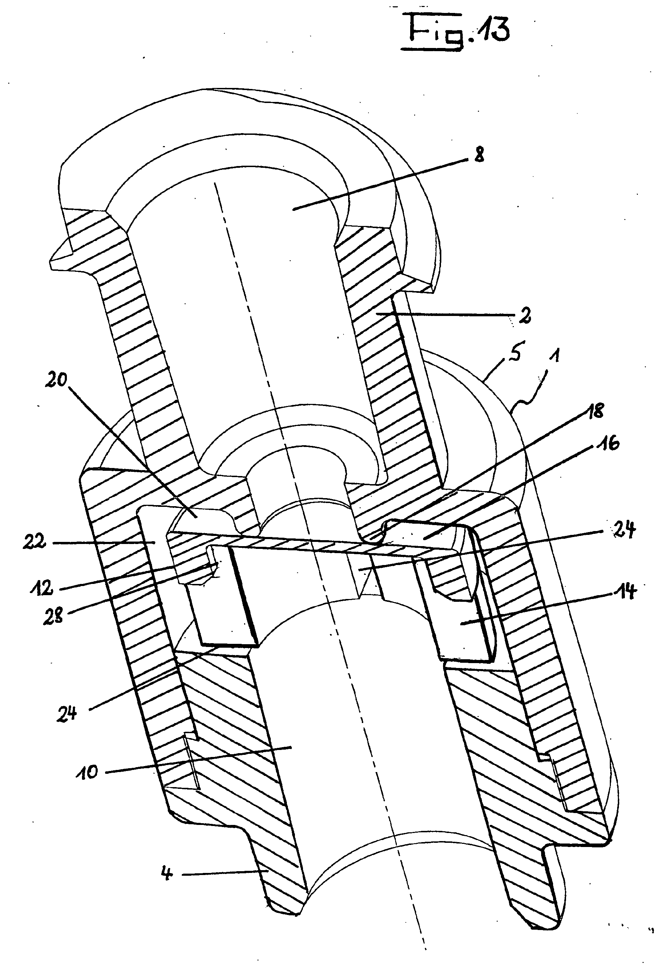

Die Figuren 13 bis 15 zeigen weitere Ausführungsformen des Rückschlagventils nach der Erfindung, wobei hier lediglich gezeigt ist, dass sich verschiedene Anschlussvarianten des Einlasskanals 8 beziehungsweise Auslasskanal 10 ohne Schwierigkeiten verwirklichen lassen.

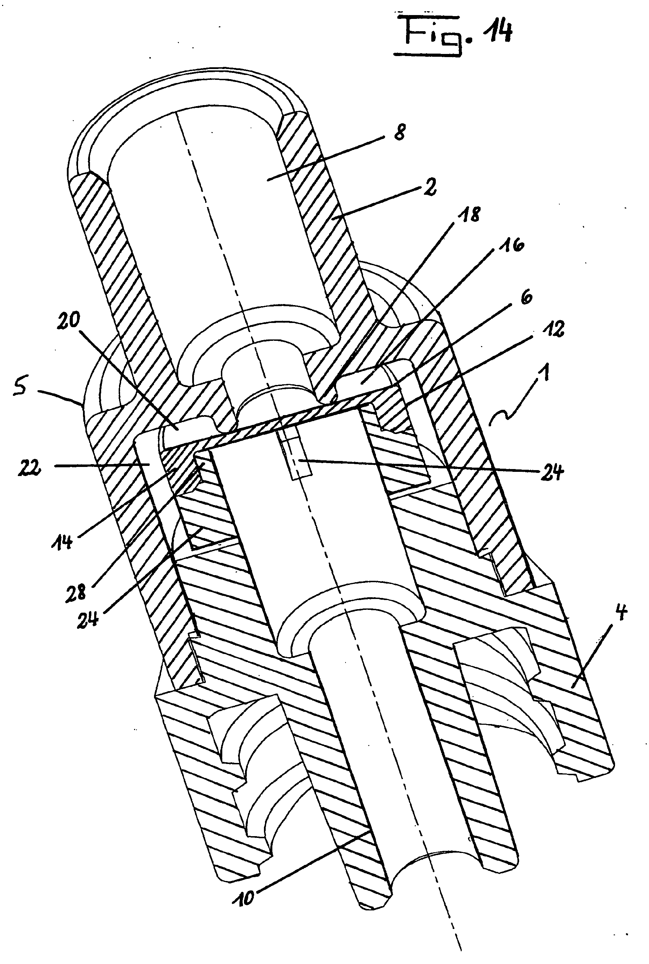

So zeigt Figur 13 einen Auslasskanal 10, welcher für einen Schlauchanschluss gedacht ist, während der Eingangskanal 8 als weiblicher Luer-Lock-Anschluss ausgebildet ist. Bei Figur 14 ist wiederum der Eingangskanal 8 als Schlauchanschluss ausgebildet, während der Ausgangskanal 10 als männlicher Luer-Lock-Anschluss ausgeformt ist.

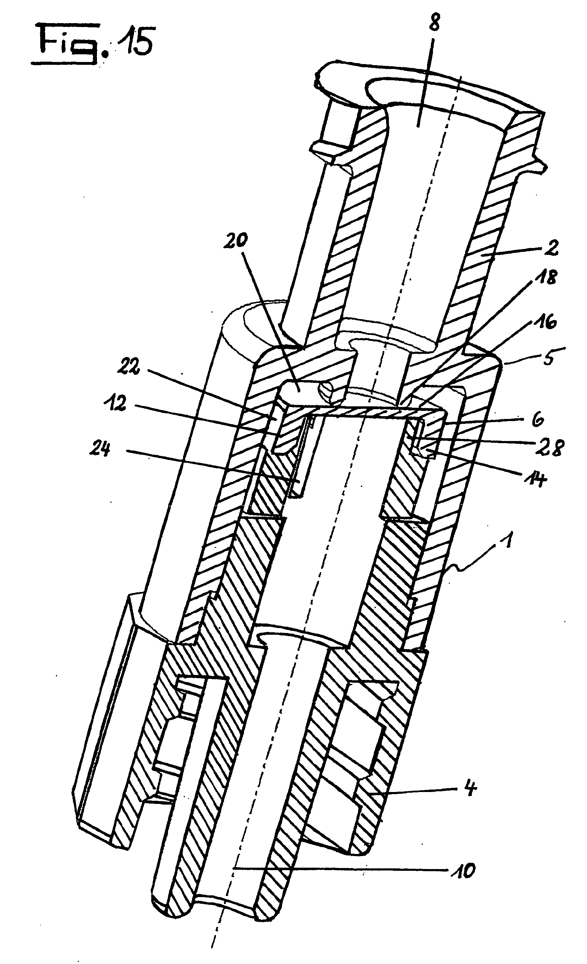

Bei dieser Ausführungsform gemäß Figur 15 ist der Eingangskanal 8 als weiblicher Luer-Lock-Anschluss ausgebildet, während der Auslasskanal 10 einen männlichen Luer-Lock-Anschluss trägt.

Sämtliche aus der Beschreibung, den Ansprüchen und den Zeichnungen hervorgehenden Merkmale und Vorteile der Erfindung., einschließlich konstruktiver Einzelheiten und räumlicher Anordnungen, können sowohl für sich als auch in beliebiger Kombination erfindungswesentlich sein.

### BEZUGSZEICHENLISTE

- 1 =: Rückschlagventil
- 2 =: Schlauchanschlussgehäuse
- 4 =: Schlauchanschlussgehäuse
- 5 =: Ventilgehäuse
- 6 =: Membrane
- 8 =: Einlasskanal
- 10 =: Auslasskanal
- 11 =: Seitenwand (zylindrisch) v. 2
- 12 =: Seitenwand v. 6
- 13 =: Stege zwischen 22
- 14 =: Unterkante v. 12
- 15 =: Nuten
- 16 =: Boden v. 6
- 17 =: Materialstreifen
- 18 =: Ventilsitz
- 19 =: Bohrung
- 20 =: ringförmiger Ventilraum
- 22 =: Bypasskanäle
- 24 =: Verbindungskanal
- 25 =: zweiter Ringraum
- 26 =: kreisförmiger Ventilraum
- 28 =: Stützwand
- 29 =: Ringschulter
- 30 =: Verdickung
- 32 =: Verbindungsabschnitt

## Patentansprüche

1. Rückschlagventil (1), insbesondere für die Medizintechnik, mit einem ersten und einem, mit diesem verbundenen zweiten Schlauchanschlussgehäuse (2, 4) und mit einer zwischen den beiden Schlauchanschlussgehäusen (2, 4) angeordneten Membrane (6) aus einem flexiblen Werkstoff, welche gegen einen ringförmigen Ventilsitz (18) vorgespannt ist und welche bei Überdruck in einem Eingangskanal (8) des ersten Schlauchanschlussgehäuses (2) unter Freigabe eines Durchflussquerschnittes von dem Ventilsitz (18) abhebbar ist und bei Überdruck in einem Ausgangskanal (10) des zweiten Schlauchanschlussgehäuses (4) zum Schließen sicher und in Minimalzeiten auf den Ventilsitz (18) andrückbar ist, wobei die Membran (6) durchgehend ohne Öffnungen ausgebildet ist und auf beiden Seiten der Membran (6) je ein ringförmiger bzw. kreisförmiger Ventilraum (20, 26) vorgesehen ist, wobei der ringförmige Ventilraum (20) den Ventilsitz (18) umgibt und die beiden Ventilräume (20, 26) durch Bypasskanäle (22, 24) miteinander verbunden sind, **dadurch gekennzeichnet, dass** die Membran (6) topfartig mit gegenüber ihrem Boden (16) verstärkter Seitenwand (12) ausgebildet ist, dass die Membran (6) mit ihrer Seitenwand (12) unter Vorspannung über eine ringförmige Stützwand (28) geschoben ist, und dass der ringförmige Ventilsitz (18) radial innerhalb der Stützwand (28) angeordnet ist und axial der Stützwand (28) gegenüberliegend auf der Außenseite des Bodens (16) anliegt..

2. Rückschlagventil nach Anspruch 1, **dadurch gekennzeichnet, dass** die Bypasskanäle (22) zwischen der Seitenwand (12) der Membran (6) und dem zweiten Schlauchanschlussgehäuse (4) vorgesehen sind.

3. Rückschlagventil nach Anspruch 2, **dadurch gekennzeichnet, dass** die Bypasskanäle (22) in dem zweiten Schlauchanschlussgehäuse (4) axial zur Seitenwand (12) verlaufend ausgebildet sind.

4. Rückschlagventil nach Anspruch 2, **dadurch gekennzeichnet, dass** die Bypasskanäle (22) in der Seitenwand (12) der Membran (6) ausgebildet sind.

5. Rückschlagventil nach einem der vorstehenden Ansprüche, **dadurch gekennzeichnet, dass** der den Ventilsitz (18) umgebende Ringraum (20) auf der Außenseite des Bodens (16) über die Bypasskanäle (22, 24) mit dem Auslasskanal (10) des Rückschlagventils (1) verbunden ist.

6. Rückschlagventil nach einem der vorstehenden Ansprüche, **dadurch gekennzeichnet, dass** die Stützwand (28) mit dem zweiten Schlauchanschlussgehäuse (4) einstückig ausgebildet ist.

7. Rückschlagventil nach einem der vorstehenden Ansprüche, **dadurch gekennzeichnet, dass** die Schlauchanschlussgehäuse (2, 4) tief ineinander geschachtelt ausgebildet sind, derart, dass der Ventilsitz (18) tief innerhalb des ersten Schlauchanschlussgehäuses (2) liegt.

8. Rückschlagventil nach einem der vorstehenden Ansprüche, **dadurch gekennzeichnet, dass** die Membran (6) aus einem thermoplastischen Elastomer besteht.

## Claims

1. Check valve (1), especially for the medical technique, having a first and a second hose connector housing (2, 4) joined with each other, and a diaphragm (6) of a flexible material positioned between the two hose connector housings (2, 4) which is pretensioned against an annular valve seat (18) and which at an over-pressure in an entry channel (8) of the first hose connector housing (2) can be lifted from the valve seat (18) opening a flow cross-section and which at an over-pressure in an outlet channel (10) of the second hose connector housing (4) can be pressed onto the valve seat (18) safely and in minimal times for closing the valve, wherein the diaphragm (6) is solid without any openings and, wherein on both sides of the diaphragm (6) each an annular or circular valve space (20, 26), respectively, is provided, wherein the annular valve space (20) is surrounding the valve seat (18) and, wherein the two valve spaces (20, 26) are connected by bypass channels (22, 24) with each other, **characterised in that** the diaphragm (6) is cup-shaped and having an increased side wall (12) compared with its bottom (16), that the diaphragm (6) is pushed under pretension over an annular supporting wall (28) with its side wall (12) and that the annular valve seat (18) is positioned radially inwardly of the supporting wall (28) and axially opposite to the supporting wall (28) is contacting the outside of the bottom (16).

2. Check valve according to claim 1, **characterised in that** the bypass channels (22) are provided between the side wall (12) of the diaphragm (6) and the first hose connector housing (4).

3. Check valve according to claim 2, **characterised in that** the bypass channels (22) are provided in the second hose connector housing (4) extending axially with respect to the side wall (12).

4. Check valve according to claim 2, **characterised in that** the bypass channels (22) are provided in the side wall (12) of the diaphragm (6).

5. Check valve according to any of the preceding claims, **characterised in that** the annular space (20) surrounding the valve seat (18) on the exterior of the bottom (12) is connected by bypass channels (22, 24) with the outlet channel (10) of the check valve (1).

6. Check valve according to any of the preceding claims, **characterised in that** the supporting wall (28) is monolithic with the second hose connector housing (4).

7. Check valve according to any of the preceding claims, **characterised in that** the hose connector housing (2, 4) are deeply fitted into one another such that the valve seat (18) deeply is positioned within the first hose connector housing (2).

8. Check valve according to any of the preceding claims, **characterised in that** the diaphragm (6) is consisting of a thermoplastic elastomer.

## Revendications

1. Clapet anti-retour (1), en particulier pour la technique médicale, comprenant un premier, et un deuxième boîtier de raccord de flexible (2, 4) connecté au premier, et comprenant une membrane (6) en matériau flexible disposée entre les deux boîtiers de raccord de flexible (2, 4), laquelle est précontrainte contre un siège de clapet de forme annulaire (18) et peut être soulevée du siège de clapet (18) en cas de surpression dans un canal d'entrée (8) du premier boîtier de raccord de flexible (2) en libérant une section transversale d'écoulement, et en cas de surpression dans un canal de sortie (10) du deuxième boîtier de raccord de flexible (4) peut être pressée fermement et en un temps minimal sur le siège de clapet (18) pour assurer la fermeture, la membrane (6) étant réalisée entièrement sans ouverture et étant pourvue sur les deux côtés de la membrane (6) d'un espace de clapet respectif annulaire ou circulaire (20, 26), l'espace de clapet annulaire (20) entourant le siège de clapet (18) et les deux espaces de clapet (20, 26) étant connectés l'un à l'autre par des canaux de dérivation (22, 24), **caractérisé en ce que** la membrane (6) est réalisée en forme de pot avec une paroi latérale (12) renforcée par rapport à son fond (16), **en ce que** la membrane (6) est poussée avec sa paroi latérale (12) avec précontrainte par-dessus une paroi de support annulaire (28) et **en ce que** le siège de clapet annulaire (18) est disposé radialement à l'intérieur de la paroi de support (28) et s'applique axialement en regard de la paroi de support (28) sur le côté extérieur du fond (16).

2. Clapet anti-retour selon la revendication 1, **caractérisé en ce que** les canaux de dérivation (22) sont prévus entre la paroi latérale (12) de la membrane (6) et le deuxième boîtier de raccord de flexible (4).

3. Clapet anti-retour selon la revendication 2, **caractérisé en ce que** les canaux de dérivation (22) sont réalisés de manière à s'étendre axialement par rapport à la paroi latérale (12) dans le deuxième boîtier de raccord de flexible (4).

4. Clapet anti-retour selon la revendication 2, **caractérisé en ce que** les canaux de dérivation (22) sont réalisés dans la paroi latérale (12) de la membrane (6).

5. Clapet anti-retour selon l'une quelconque des revendications précédentes, **caractérisé en ce que** l'espace annulaire (20) entourant le siège de clapet (18) est connecté du côté extérieur du fond (16) par le biais des canaux de dérivation (22, 24) au canal de sortie (10) du clapet anti-retour (1).

6. Clapet anti-retour selon l'une quelconque des revendications précédentes, **caractérisé en ce que** la paroi de support (28) est réalisée d'une seule pièce avec le deuxième boîtier de raccord de flexible (4).

7. Clapet anti-retour selon l'une quelconque des revendications précédentes, **caractérisé en ce que** les boîtiers de raccord de flexible (2, 4) sont réalisés de manière profondément emboîtés l'un dans l'autre de telle sorte que le siège de clapet (18) se trouve profondément à l'intérieur du premier boîtier de raccord de flexible (2).

8. Clapet anti-retour selon l'une quelconque des revendications précédentes, **caractérisé en ce que** la membrane (6) se compose d'un élastomère thermoplastique.
